# EUROPEAN PATENT SPECIFICATION

(11) **EP 0 514 465 B1**
(45) Date of publication and mention of the grant of the patent: **23.04.1997**
(21) Application number: 91904472.7
(22) Date of filing: 08.02.1991
(51) Int. Cl.: A61K 9/02, A61K 9/12

(54) **FOAMABLE COMPOSITION FOR PHARMACEUTICAL USE, USE THEREOF AND METHOD OF TREATMENT**
VERSCHÄUMBARES MITTEL ZUR PHARMAZEUTISCHEN VERWENDUNG, DESSEN VERWENDUNG SOWIE BEHANDLUNGSVERFAHREN
COMPOSITION MOUSSANTE A USAGE PHARMACEUTIQUE, ET PROCEDES D'UTILISATION ET DE TRAITEMENT ASSOCIES

(30) Priority: 09.02.1990 SE 9000485
(43) Date of publication of application: 25.11.1992
(73) Proprietor: Pharmacia & Upjohn Aktiebolag, 112 87 Stockholm (SE)
(72) Inventor: CAMBER, Ola, S-756 53 Uppsala (SE); INTO-MALMBERG, Uno, S-754 40 Uppsala (SE)
(74) Representative: Widén, Björn
(86) International application number: SE9100088
(87) International publication number: WO9111991

(56) References cited:
- EP-A- 0 062 000
- EP-A- 0 395 329
- FR-A- 2 647 344
- US-A- 3 770 648
- US-A- 4 657 900
- Congr. Int. Technol. Pharm., 5th 1989, 3, p. 154-162, 5th International Conference on Pharmaceutical Technology, C.S. QIAO et al: "Realization and pharmacokinetic study of a rectal foam of paracetamol".
- CHEMICAL ABSTRACTS, Volume 103, No. 25, 23 December 1985, (Columbus, Ohio US), LYAPUNOV N.A. et al: "Biopharmaceutical studies of estrogens in the form of foaming aerosols", see page 153, Abstract 206894y, & Farmatsiya (Moscow) 1985, 34 (5), 25-29.

## Description

### Technical field of the invention

The present invention relates to the field of rectal administration of a pharmaceutical preparation. More specifically, the invention relates to the use of a foamable composition for the manufacture of a pharmaceutical preparation to be used in rectal administration as well as to the foamable composition for use as a pharmaceutical preparation for rectal administration. The invention also relates to such a pharmaceutical preparation to be used for rectal administration and to a method of its production.

Aqueous or oily foamable pharmaceutical preparations are previously known for rectal use, but the present invention is based on the use of an essentially non-aqueous and non-oily composition in connection with such a rectal application. Although non-aqueous and non-oily foamable compositions have been utilized for medical purposes, e.g. for topical applications, they have not successfully been used for rectal administrations. Therefore, the invention represents a valuable alternative to other ways of administration or a valuable improvement of previously known foamable compositions utilized in rectal administrations.

### Background of the invention

The rectal way of administering a pharmaceutical is a common alternative when an administration the oral way causes problems due to swallowing difficulties or gastro intestinal side effects. Often pharmaceuticals are administered rectally to obtain a local effect (an astringent or disinfectant effect). Furthermore, the rectal way of administering the pharmaceutical reduces its first metabolism through the liver. This means that the rectal administration of pharmaceuticals can be utilized to achieve a higher bioavailability than an administration the oral way. However, with the same dosage of pharmaceutical the absorbtion is generally slower as well as obtained to a less degree than from the tablet form. For pharmaceuticals having low water solubilities the absorbtion rate is more dependent on the area of the pharmaceutical which is dissolved than the concentration of the preparation. This means that the effects of pharmaceuticals acting locally in colon are dependent on factors such as the shape of the preparation, distal spreading and retention time in the intestines.

The rectal preparations comprise suppositories, enema (clyster, rectiol) and aqueous foamable preparations. Of these the suppository is the most common one. The suppository base is generally a fat but also water-soluble or water-miscible bases are utilized. To obtain a good bioavailability the pharmaceutical should come into contact with the intestinal mucosa. Also for a local effect this is essential. This desideratum will be fulfilled very varyingly dependent on whether the pharmaceutical is present in the dissolved, emulsified or suspended form in the suppository base. The physical properties of the pharmaceutical also play an important role. Also with reference to the second most common preparation form, enema, the above-mentioned is of great importance. This preparation form consists of a solution or suspension of the pharmaceutical. By the addition of viscosity-enhancing substances a longer rectal retention time is achieved which means an increased bioavailability of the pharmaceutical referred to.

Aqueous foamable preparations are the less common of the rectal preparation forms. The relatively complicated manufacture thereof as well as the complicated package as compared to suppositories and enema are probably major causes therefor. However, since better spreading effects are obtained with enema and foams than with suppositories more distal intestine regions can be reached thereby. Therefore, also some foam preparations for rectal use have come on the market, especially as a less amount of water is administered by means of a foam preparation than by means of enema, which means less discomfort for the patient.

As far as we know all foam preparations put on the market are aqueous and contain steroids. There are, however, a number of disadvantages associated with the use of such aqueous foams, which disadvantages are previously known or have been manifested in connection with the work leading to the present invention, as will be described more below. As concerns the prior art in this field reference is made to M L Clark, the Practitioner, Vol 219, July 1977, p 103; W S J Ruddel, R J Dickinson, M F Dixon and A T R Axon, Gut, Vol 21, 1980, No 10, p 885; The Pharmaceutical Journal, Vol 294m, March 7, 1987, p 308, 311; and US Patent No 2,729,586.

A non-aqueous foam preparation for rectal administration of paracetamol has been described by C.S. Ciao et al, "Realization and pharmacokinetic study of a rectal foam of paracetamol", 5th International Conference on Pharmaceutical Technology", Papers on 31 May 1989, afternoon. However, also this foam composition is associated with disadvantages and the purposes of the present invention are not attained (see comparative example below). It is further to be noted that this foam apparently is to be administered in pre-expanded form from a rectal applicator.

Thus, the basis for the present invention is the discovery that by using an essentially non-aqueous and non-oily foamable composition for the manufacture of a pharmaceutical preparation to be used for rectal administration of the pharmaceutically active ingredient and selecting the ingredients and the proportions thereof properly, great advantages can be obtained as compared to the use of aqueous foamable compositions for such an administration, in addition to those advantages which are obtained by the rectal way of administration as compared to other routes for the administration referred to.

### General disclosure of the invention

In accordance with the present invention it has unexpectedly been found that an essentially non-aqueous and non-oily foamable composition can be used for rectal administration of pharmaceuticals while achieving several advantages as compared to the use of an aqueous or oily foamable composition for a similar administration.

Therefore, a primary object of the invention is to provide the use of an essentially or completely non-aqueous foamable composition for the manufacture of a pharmaceutical preparation to be used for rectal administration thereof.

Another object of the invention is to provide the use of said aqueous foamable composition for the manufacture of a pharmaceutical preparation, the foaming of which is delayed, or non-immediate, when administered rectally, which is a valuable contribution to the technique of using aqueous foams where an immediate foaming effect is obtained.

One advantage associated with the delayed foaming is that no defecation reflex is induced. Another object of the invention therefore is to provide the use of the new non-aqueous foamable composition for the manufacture of a pharmaceutical preparation which induces no or essentially no defecation reflex when administered rectally.

The delayed foaming effect also means that a better spreading effect, or bioavailability, is obtained as compared to the momentaneous foaming hitherto obtained. Therefore, still another object of the invention is to provide the use of the above-mentioned non-aqueous foamable composition for the manufacture of a pharmaceutical preparation giving an outstanding spreading effect or bioavailability in rectal administration.

Still another object of the invention is to provide the use of the non-aqueous foamable composition referred to for the manufacture of a pharmaceutical preparation useful for moisture- or water-sensible substances in rectal administration thereof, which substances are not rectally administerable by means of aqueous foamable compositions.

A still further object of the invention is to provide the use of said non-aqueous foamable composition for the manufacture of a pharmaceutical preparation having superior adhesive properties relative to the intestines or mucosas as compared to the corresponding properties of aqueous compositions. Thereby a delayed contact or retention time as concerns the interaction between pharmaceutical substance and site of action, i.e. a good bioavailability, is obtained. Said outstanding adhesive properties also influence upon the defecation reflex referred to above, which means an even less defecation reflex and an excellent compliance for the patient.

Another object of the invention is to provide the use of the foamable composition for the manufacture of a pharmaceutical preparation, the foaming action of which is reproducible and reliable, i.e. allowing a controlled release of the pharmaceutical to be used substantially independently of the nature of the pharmaceutical.

According to another aspect of the invention a foamable composition is provided for use as a pharmaceutical preparation for rectal administration of a pharmaceutically active ingredient, said foamable composition showing the advantages referred to.

According to still another aspect of the invention a pharmaceutical preparation to be used for rectal administration is provided which comprises a foamable composition showing the features and advantages referred to.

According to a further aspect of the invention a method is provided for producing a pharmaceutical preparation to be used for rectal administration and comprising a foamable composition showing the features and advantages referred to.

These and other objects and advantages of the present invention will become apparent from the following more detailed description of the invention.

### Detailed disclosure of the invention

In accordance with the invention some or all of the above-mentioned objects and other objects are accomplished by the provision of a use of an essentially or completely non-aqueous foamable composition for the manufacture of a pharmaceutical preparation to be used in a pressurized container of the aerosol type for rectal administration of said pharmaceutical preparation, said foamable composition comprising a liquid polar polyol or polyol mixture, optionally in admixture with a minor amount of water, a pharmaceutically active ingredient, at least one foam-stabilizing and emulsifying surfactant and optionally minor amounts of conventional additives for pharmaceutical compositions, all ingredients being pharmaceutically acceptable and being mixed in such proportions and with such an amount of a propellant having such a vapour pressure that a non-immediate, or delayed, foaming action is obtained, i.e. that only a partial foaming action is obtained initially, when the pharmaceutical preparation is caused to foam or is rectally administered.

Thus, generally the major aspect or discovery in connection with the invention is that the previously used aqueous foamable compositions can be replaced by similar essentially non-aqueous foamable compositions for rectal administration of pharmaceuticals. More specifically, it has unexpectedly been shown that in order to have bioavailability of the pharmaceutical in the intestines the pharmaceutical preparation need not be aqueous and thereby compatible with the gastric juice. In addition thereto, it has surprisingly been discovered that superior pharmaceutical and compliance effects are obtained when utilizing essentially non-aqueous foamable compositions in a new way, i.e. by rectal administration thereof, as compared to the rectal administration of aqueous foamable compositions.

Although such advantages have been referred to above, a summary of the most important ones is as follows. The essentially non-aqueous foamable compositions used according to the present invention possess a delayed foaming action which means that a far better foaming, viz. up in the intestines, is obtained. A better spreading effect is thereby obtained, which is useful for instance when treating distally localized ulcerative colitis and similar diseases. Furthermore, it has been found that the foaming effect when using essentially non-aqueous compositions in rectum is much more reproducible or controllable than when using aqueous compositions. Therefore, a very reliable and controllable drug delivery system is accomplished by means of the present invention.

As the compositions utilized in the present invention are essentially non-aqueous they can be utilized for the delivery of drugs or pharmaceuticals which are water-sensitive, for instance for rectal administration of pharmaceuticals which cannot be orally administered due to the fact that they are destroyed by the gastric juices.

The adhesive properties as to the contact between the foam and the intestinal mucosa are excellent. This means that a superior bioavailability is obtained, and in addition thereto the defecation reflex is not induced when the essentially non-aqueous compositions are used in accordance with the present invention for the preparation of rectally administerable drugs.

From the above-mentioned it can be gathered that the major aspect of the invention is the use of an essentially non-aqueous foamable composition. More specifically this means that the composition is based on a liquid polar polyol or polyol mixture as was mentioned above. In this context the term polar should be interpreted in the common sense, i.e. water-soluble or water-miscible. The term liquid means present in the liquid state at around room or ambient temperature. With reference to the expression essentially or completely non-aqueous in connection with the foamable composition it is to be understood that generally water is to be excluded from the composition but that minor amounts thereof may be present as long as they do not have any essential negative effect on the properties referred to and as it is generally difficult to exclude water up to 100 %. In other words, generally up to 30 %, preferably up to 20 %, and most preferably up to 3 %, of water can be accepted for most purposes of the invention, said percentages being based on the total weight of the polyol plus water.

As can be gathered from the more detailed disclosure below with reference to the polyol, said term polyol includes dihydroxy as well polyhydroxy compounds.

Furthermore, as has already been mentioned above, the polyol should be a liquid, which in practice means a liquid already at room temperature to enable an easy preparation of the foamable composition and at least up to the temperature in rectum to enable a proper foaming effect in the rectal administration. In general the term polar means any polar liquid other than water and fulfilling the other requirements according to the invention, i.e. a polar liquid that is water-soluble or water-miscible, so as to accomplish a good bioavailability of the pharmaceutical ingredient(s).

However, an especially preferable group of polyols for use in accordance with the present invention is the group consisting of polyalkylene glycols, alkylene glycols and glycerol. Preferably, the polyol is a polyalkylene glycol or glycerol, and more preferably a polyalkylene glycol. Said polyalkylene glycol is preferably a polyethylene glycol, and with reference to the polyalkylene glycols in general as well as the especially preferable polyethylene glycols a preferable average molecular weight is 300-800, especially 400-700. Typical examples of preferable polyethylene glycols are polyethylene glycol 600 and polyethylene glycol 400, especially as said specific polyethylene glycols are available on the market in pharmaceutically acceptable forms, which for instance means that their contents of monoethylene glycol and diethylene glycol as well as of ethylene oxide are very minor. Thus, as is obvious to the skilled artisan, the polyol as well as all other ingredients of the composition used according to the invention have to be pharmaceutically acceptable in accordance with common practice. As to the alkylene glycols fulfilling the requirements according to the invention a preferable alkylene glycol is propylene glycol.

The new use according to the present invention is generally applicable to the administration of any pharmaceutically active ingredient for which any rectal activity can be expected. However, the invention is especially useful for rectal treatment of ulcerative colitis and in particular for distally localized colitis, thanks to the very extended foaming effect of the composition, i.e. as concerns time extension as well as length extension. A preferable group of pharmaceutically active ingredients for use in accordance with the invention is the group consisting of 4-aminosalicylic acid (4-ASA) and 5-aminosalicylic acid (5-ASA) as well as pharmaceutically acceptable salts thereof, such as the sodium salts; salicylazosulfapyridine (salazopyrine); and steroids having anti-inflammatory actions, such as hydrocortisone, prednisolone, budesonide; and local anaesthetics. Not only substances having a local effect in the rectum and distal colon, but also other pharmaceutically active substances may advantageously be administered in accordance with the invention. Exemplary are substances which are metabolized to a great extent (e.g. 90 % or more) at first bypass in the liver, such as analgetics, e.g. morphine, codeine; substances for systemic effect which decompose through the acidity of gastric juice or by the gastro-intestinal enzymes, such as peptides, e.g. IGF-I, IGF-II, EGF, insulin; or proteins, e.g. enzymes; and substances which are hydrolyzed in an aqueous environment.

With reference to the surfactant or mixture of surfactants to be used in the present invention it can be chosen according to principles which are known per se now that the new inventive idea has been presented. That is, said surfactant or surfactant mixture is selected so as to give a good foaming effect with reference to the polyol used as well as a good emulsifying effect with reference to the ingredients to be emulsified in the composition. Therefore, in general said surfactant or surfactants can be selected in accordance with known principles. Thus, to obtain the desired delayed expansion of the foam the propellant used must be emulsified into the liquid phase in such a manner that it is dispersed and strongly bound in the composition so as to permit it to be released for a longer period of time than is the case of conventional foam formulations. It must simultaneously be ensured that the foam obtains such a stability that it will not be broken down during the expansion phase. By selecting the proportion of surfactant or surfactants properly it may also be ensured that a high concentration of solid pharmaceutically active substance will not disturb the foam formation by causing undesired release of propellant. It will normally be necessary to use two or more different surfactants. In such a mixture of surfactants one (or more) may substantially provide the foam stabilizing activity, whereas the other (or others) may provide the emulsifying activity. However, said surfactant or surfactants are preferably selected from non-ionic surfactants, and especially preferable embodiments of such non-ionic surfactants are liquid or waxy polyoxyethylene-fatty alcohol ethers, polyoxyethylene-fatty acid esters, sorbitol-fatty acid esters, POE-sorbitol-fatty acid esters as well as self-emulsifying waxes. Specific examples of such surfactants are: sorbitol-monolaurate (Span 20), sorbitol-monooleate (Span 80), sorbitol-monopalmitate (Span 40), sorbitol-monostearate (Span 60), POE(23)-laurylether (Brij 35), POE(2)-stearylether (Brij 72), POE(10)-oleylether (Brij 96), POE(21)-stearylether (Brij 721), POE(20)-cetostearylether, POE(35)-castor oil, POE(40)-hydrogenated castor oil, POE(40)-stearic acid ester (Myrj 52), POE(50)-stearic acid ester (Myrj 53), POE(20)-sorbitol-monolaurate (Tween 20), POE(20)-sorbitol-monostearate (Tween 60), POE(20)-sorbitol-monooleate (Tween 80), POE(40)-sorbitol-septaoleate and Emulsifying wax NF and Polawax A-31. Here POE represents polyoxyethylene while Span, Brij, Myrj, Tween, Emulsifying wax NF and Polawax A are trade names which may be registered trade marks in some countries.

The composition can of course also contain conventional additives in accordance with the known technique. That is, any common adjuvant or additive can be added in a conventional amount, i.e. a minor amount not affecting the activities aimed at, for any conventional purpose. An example of such an additive is an anti-oxidant, which should for instance be used when such oxidation-sensitive substances like 4-aminosalicylic acid or 5-aminosalicylic acid are utilized.

Finally, a propellant is to be used to accomplish the necessary foaming effect. The choice thereof can also be made according to known principles for the preparation of a foamable composition packed in a pressurized container, e.g. of the aerosol type, and dispensed in rectum by a vaporization of said propellant to accomplish the foaming of the composition therein. Preferably, however, the propellant should have a vapour pressure at about room or ambient temperature within the range of 0.8-2.5 bars, especially 1.0-2.5 bars.

Furthermore, it is advisable to utilize a propellant having a boiling point around or rather close to 0°C, which is typically from -10°C to +10°C. According to one embodiment thereof said propellant is selected from the group consisting of alkanes and chlorinated-fluorinated alkanes, e.g. CClF₂-CClF₂, CH₃-CClF₂, C₄H₁₀ and C₃H₈ + C₄H₁₀.

In this context it could be added that CClF₂-CClF₂ has a boiling point of 3.6°C and a vapour pressure of 0.91 bars at 21°C, while CH₃-CClF₂ boils at -9.4°C and has a vapour pressure of 2.05 bars (21°C) and C₄H₁₀ boils at -2.2°C and has a vapour pressure of 0.20 bars (21°C). The boiling point for said mixture of C₃H₈ + C₄H₁₀ is of course not any exactly defined boiling point, but the vapour pressure of said mixture at 21°C is 2.20 bars.

From the environmental point of view a non-fluoro-chlorohydrocarbon propellant is, of course, preferred. Suitable propellants in this respect are therefore hydrocarbon propellants, such as n-butane or a mixture of propane and iso-butane.

As was mentioned above, the composition used according to the present invention is preferably packed in a conventional pressurized container of the aerosol type, and as concerns such containers and the technique of preparing them reference is made to conventional technique.

Now that the general inventive concept has been disclosed, the exact proportions between the ingredients as well as the specific choice of propellant gas can easily be made by the skilled artisan while considering the foaming effect required or desired. Therefore, the ingredients are mixed in such proportions that a delayed foaming effect is obtained as compared to the case where the base is water rather than the polyol used in accordance with the present invention. In this context, the term delayed foaming action is not any exact or critical expression but is generally selected by a person skilled in the art for each specific case. Generally, however, such proportions of the ingredients shall be utilized that less than 50 percent by volume of the totally obtainable foaming effect is obtained during the first second of the foaming. Preferably less than 50 percent by volume of the totally obtainable foaming effect should be obtained during the first 2, and more preferably during the first 5 seconds of the foaming, and most preferably less than 50 percent by volume of the totally obtainable foaming effect should be obtained during the first 10 seconds of the foaming. Suitably, complete expansion is obtained within no more than about 2 minutes. Although not quite comparable with the foaming in rectum, said foaming effect could typically be ascertained by a simple experimentation performed in vitro, i.e. by measuring the total expansion of the foam while monitoring said expansion versus the time. In contrast immediate foaming means a total expansion in, say, about, the order of a tenth of a second.

Moreover, it can be added that generally or according to a preferable embodiment of the invention the composition should be such that the foam expands to a volume that is approximately 15-20 times the volume of the original or non-expanded foamable composition. Thus, for instance, 10 mls of a concentrate should give 150-200 mls of the foam.

According to a preferable embodiment of the invention the pharmaceutical preparation comprises 60-95, preferably 70-93, percent by weight of the polyol plus water if present; up to 30, preferably 0.001-25, percent by weight of the pharmaceutically active ingredient; 0.2-10, preferably 0.5-8, percent by weight of the surfactant; 0-5, preferably 0-2, percent by weight of the conventional additive; and 1-15, preferably 2-10, percent by weight of the propellant, all percentages being based on the total weight of the pharmaceutical preparation.

According to one important embodiment within the above-mentioned composition, where the amount of the pharmaceutically active ingredient is "high", said pharmaceutical preparation comprises 70-89 percent by weight of the polyol plus water if present; 5-25 percent by weight of the pharmaceutically active ingredient; 0.8-6 percent by weight of the surfactant; 0-2 percent by weight of the conventional additive; and 2-10 percent by weight of the propellant.

According to another embodiment of the invention, where the amount of the pharmaceutically active ingredient is "low", said pharmaceutical preparation preferably comprises 89-93 percent by weight of the polyol plus water if present; 0.001-25, e.g. 0.5-5 percent by weight of the pharmaceutically active ingredient; 0.8-6 percent by weight of the surfactant; 0-2 percent by weight of the conventional additive; and 2-8 percent by weight of the propellant.

The foamable composition as well as the pharmaceutical preparation used according to the present invention can be manufactured according to known techniques for the manufacture of foamable compositions for use in the pressurized containers. A typical example of a suitable manufacturing process is, however, as follows.

The polyol and the surfactant are heated together during stirring of the mixture so as to obtain a liquefaction or melting of the surfactant. This is accomplished so as to obtain an emulsion that can be homogenized to obtain a good degree of dispersability.

Optional additives, e.g. an anti-oxidant, can now be added. The emulsion is then allowed to cool to any proper temperature, typically 25-30°C, with continued stirring thereof.

Then the pharmaceutically active ingredient is suspended or dissolved in the emulsion. The foam or foamable base now obtained is filled into an aerosol container in such an amount that the propellant gas can also be filled into said container while still maintaining an empty space within said container to be used for shaking said gas into the foamable base.

As was mentioned above the invention also relates to the essentially or completely non-aqueous foamable composition as defined above, for use as a pharmaceutical preparation for rectal administration of the pharmaceutically active ingredient, especially for rectal treatment of ulcerative colitis, as well as such a pharmaceutical preparation per se and its production. Thus, all essential and preferable characteristics of the foamable composition per se have been described above in connection with the use referred to and need not be repeated here.

### EXAMPLES

The invention will now be further described by means of the following non-limiting examples.

The compositions 1-7 identified below were manufactured in the following way.

The polyols and surfactants were heated during stirring so as to obtain a melting of the surfactants. The additives were also added during this operation. The emulsion formed was allowed to cool to about 25-30°C with continued stirring.

Then, the active pharmaceutical ingredients were suspended or dissolved in the suspension and the foamable bases thus obtained were filled into aerosol cans in accordance with conventional practice.

Compositions 1-7 were as follows.
1.

| | |
|---|---|
| PEG 600 | 82 grams |
| Brij 72 | 0.6 grams |
| Brij 96 | 0.4 grams |
| Sodium ascorbate | 1.0 grams |
| 4-ASA-sodium salt | 27.6 grams |
| n-Butane | 6 grams |

2.

| | |
|---|---|
| PEG 600 | 82 grams |
| Polawax A-31 | 1.0 grams |
| Brij 721 | 1.0 grams |
| Sodium ascorbate | 1.0 grams |
| 4-ASA-sodium salt | 27.6 grams |
| n-Butane | 6 grams |

3.

| | |
|---|---|
| PEG 600 | 60 grams |
| Glycerol | 24.6 grams |
| Brij 72 | 1.0 grams |
| Arlatone T | 0.6 grams |
| Sodium ascorbate | 1.0 grams |
| 4-ASA-sodium salt | 27.6 grams |
| n-Butane | 6 grams |

4.

| | |
|---|---|
| PEG 600 | 60 grams |
| Glycerol | 24.6 grams |
| Polawax A-31 | 2.0 grams |
| Brij 721 | 2.0 grams |
| Sodium ascorbate | 1.0 grams |
| 4-ASA-sodium salt | 27.6 grams |
| n-Butane | 6 grams |

5.

| | |
|---|---|
| PEG 400 | 82 grams |
| Polawax A-31 | 2.0 grams |
| Brij 721 | 1.0 grams |
| Sodium ascorbate | 1.0 grams |
| 4-ASA-sodium salt | 27.6 grams |
| n-Butane | 6 grams |

6.

| | |
|---|---|
| PEG 400 | 82 grams |
| Polawax A-31 | 1.0 grams |
| Brij 72 | 0.5 grams |
| Brij 96 | 0.5 grams |
| Sodium ascorbate | 1.0 grams |
| 4-ASA-sodium salt | 27.6 grams |
| n-Butane | 6 grams |

7.

| | |
|---|---|
| PEG 400 | 82 grams |
| Polawax A-31 | 2.0 grams |
| Brij 35 | 1.0 grams |
| Sodium ascorbate | 1.0 grams |
| 4-ASA-sodium salt | 27.6 grams |
| n-Butane | 6 grams |

The meanings of the abbreviations and trade names used are as identified above.

### Example 8

A 5-ASA rectal foam preparation having the following composition was prepared as above:

| | |
|---|---|
| Mezalazine (5-ASA) | 18.00 grams |
| Polyethylene Glycol 400 | 70.20 grams |
| Ascorbic Acid | 0.90 grams |
| Emulsifying Wax NF | 1.80 grams |
| Polyoxyl 20 (Ceto)stearyl Ether | 1.35 grams |
| Sorbitan Monostearate | 2.70 grams |
| Polysorbate 60 | 0.90 grams |
| n-Butane | 5.11 grams |

The total volume was about 90 mls. (The greatest contribution to foam stability is provided by Emulsifying Wax NF and Polyoxyl 20 (ceto)-stearyl ether.)

Nitrogen was added to a pressure of 7 bars in the aerosol can. It is to be noted, however, that the nitrogen gas does not form part of the foam base as it does not dissolve in the composition but only has the purpose of maintaining the pressure within the aerosol can at such a level that the dosage accuracy may be ensured during the time it will take to use the whole content of the can package.

The above described foam composition was tested for foaming effect by injecting it into a dialysis tube having a diameter of 1 inch and measuring the expanded foam length versus time. It was found that the composition expanded to a length of 7 cm after 1.5 sec, to 14 cm after 10 sec, and to the total foam length of 16 cm after 30-60 sec. The foam remained stable, i.e. was not broken down for 4-5 hours.

A corresponding composition prepared as above but without the mezalazine ingredient gave substantially the same results.

### Comparative example

A prior art non-aqueous foam for rectal administration of paracetamol disclosed in the previously mentioned reference Qiao et al, 1989 and consisting of 77.5 % (w/w) propylene glycol, 1.5 % (w/w) Emulsifying wax, 6 % (w/w) polysorbate 60, 15 % (w/w) paracetamol, and 10 % Freon® (12/114, 40:60) as propellant was prepared as described in the reference. Testing of the foaming effect as above revealed that this foam composition had an immediate foaming action, i.e. that expansion to full length of about 16 cm was completed in about 0.1 sec. It also showed a poor stability and was broken down in about half an hour.

A corresponding foam composition prepared without the paracetamol ingredient gave a very bad foaming indicicating that the presence of paracetamol is critical for obtaining a foam.

Substitution of mezalazine (5-ASA) for paracetamol in the above composition also gave a very poor foaming.

A bad foaming activity was similarly obtained when the Freon® propellant was replaced by n-butane.

### Examples 9-11

Experiments concerning foaming of compositions according to the invention as well as of aqueous compositions according to the prior art in recta of rabbits were performed in the following way.

### MATERIALS AND METHODS

### Animals

Female New Zealand White rabbits, weight of ≈4 kg, kept according to GLP conditions (1).

### Anesthetic

Alphaxalone/alphadolone acetate (Saffan™, Glaxovet Ltd., England).

### Chemicals

Fluorescein sodium (Gurr Ltd, England).

### Pharmacons

5-aminosalicylic acid (5-ASA) and the sodium salt of p-amino salicylic acid (Na-pAS) (Pharmacia LEO Therapeutics AB, Uppsala, Sweden).

### Experimental procedure

The rabbits were divided into groups of 3 animals for each foam preparation to be tested. The experiments were performed on the same time of the day, viz. in the afternoon. The rabbits were anesthetized (2), and placed in a supine position. 1.5 ml of a concentrate of the rectal foam was introduced into the colon about 4 cm from anus, which was then closed with a soft clip.

To estimate intraluminal spreading in the colon, 1 % of fluorescein sodium was added to the rectal foams at preparation. This spreading was measured macroscopically with the aid of a UV light source.

After 30 minutes from administration, laparotomy was performed and the colon was removed. The colon was opened longitudinally and the extent of spreading was measured with a ruler.

### Foamable compositions used

The following compositions 9-11 were used.

### 9. 5-ASA in a PEG (polyethyleneglycol) base

| | active (grams) | placebo (grams) |
|---|---|---|
| PEG 400 | 127.5 | 145.5 |
| Brij 72 | 1.5 | 1.5 |
| Brij 721 | 1.5 | 1.5 |
| Sodium ascorbate | 1.5 | 1.5 |
| 5-ASA | 30 | - |
| Propellant gas | q.s. | q.s. |

### 10. 5-ASA in an aqueous base

| | active (grams) | placebo (grams) |
|---|---|---|
| Distilled water | 121.5 | 135 |
| Self emulsifying wax | 3.0 | 3.0 |
| Brij 721 | 3.0 | 3.0 |
| Sodium ascorbate | 1.5 | 1.5 |
| 5-ASA | 30 | - |
| Hydroxypropyl cellulose | - | 3.0 |
| Propellant gas | q.s. | q.s. |

### 11. Na-PAS in a PEG base

| | active (grams) | placebo (grams) |
|---|---|---|
| PEG 400 | 123 | 147 |
| Self emulsifying wax | 3.0 | 3.0 |
| Brij 721 | 1.5 | 1.8 |
| Brij 72 | - | 0.9 |
| Sodium ascorbate | 1.5 | 1.5 |
| Na-PAS | 41.4 | - |
| Propellant gas | q.s. | q.s. |

As to the different amounts of the polyol and water, respectively, in active and placebo compositions, respectively, it should be noted that all compositions were adjusted to similar volumes, i.e. with and without active ingredients.

### RESULTS

The results of the experiments are summarized in the following table.

**Table**

| Spreadings of the foams (mean values in cm) | | |
|---|---|---|
| | active | placebo |
| Ex 9 | 30.7 | 31.1 |
| Ex 10 | 17.2 | 15.6 |
| Ex 11 | 22.7 | 33.5 |

Thus, it can be seen that the spreadings of the foams varied considerably with different foam compositions. The shortest spreadings were obtained with the aqueous based foams (Ex 10). Similar 5-ASA foams in PEG (Ex 9) showed spreadings which were above 80 % higher and which were also very consistent between the three experiments. The Na-PAS foams (Ex 11) showed spreading heights in between those of Ex 9 and Ex 10 but still about 30 % higher than those of the aqueous based foams (which are not directly comparable to the 5-ASA foams).

### REFERENCES

1. Good Laboratory Practise, Food and Drug Administration. (1987) Federal Register 21 CFR, part 58.
2. Green, CJ. Animal anaesthesia, Laboratory animal handbooks 8 (1979) Laboratory animals Ltd., London.

## Claims

1. An essentially or completely non-aqueous foamable composition for rectal administration and comprising a liquid polar polyol or polyol mixture, a pharmaceutically active ingredient capable of exerting a local effect in rectum or the distal part of colon, at least one foam-stabilizing and emulsifying surfactant, said composition being comprised within a pressurized container together with a propellant, **wherein** the proportions of said components of the composition are mixed in such proportions and with such an amount of the propellant having such a vapour pressure that less than 50% by volume of the totally obtainable foaming effect is achieved during the first second after the composition is rectally administered from the pressurized container.

2. A composition according to claim 1, **wherein** the composition contains additives for pharmaceutical compositions, e.g. antioxidants.

3. A composition according to anyone of claims 1-2, **wherein** the water content of the composition is 0-30, preferably 0-20, and at most 0-3, % by weight of the total weight of polyol plus water.

4. A composition according to anyone of claims 1-3, **wherein** the composition comprises 60-95, preferably 70-93, % by weight of the polyol plus water; up to 30, preferably 0.001-25, % by weight of the pharmaceutically active ingredient; 0.2-10, preferably 0.5-8, % by weight of the surfactant; 0-5, preferably 0-2, % by weight of the additives; and 1-15, preferably 2-10, % by weight of the propellant, all percentages being based on the total weight of the composition including the propellant.

5. A composition according to claim 4, **wherein** the composition comprises 70-89 % by weight of the polyol plus water; 5-25 % by weight of the pharmaceutically active ingredient; 0.8-6 % by weight of the surfactant; 0-2, % by weight of the additives; and 2-10, % by weight of the propellant.

6. A composition according to claim 4, **wherein** the composition comprises 89-93 % by weight of the polyol plus water; 0.001-5 % by weight of the pharmaceutically active ingredient; 0.8-6 % by weight of the surfactant; 0-2, % by weight of the additives; and 2-8, % by weight of the propellant

7. A composition according to anyone of claims 1-6, **wherein** the proportions of the ingredients including the propellant and the vapour pressure thereof, are chosen so as to obtain less than 50% by volume of the totally obtainable forming effect during the first 5 seconds of the foaming.

8. A composition according to anyone of claims 1-7, **wherein** the polyol or polyols are selected from the group consisting of liquid polyalkylene glycols, alkylene glycols and glycerol, preferably polyalkylene glycols, especially polyethylene glycols, having an average molecular weight of 300-800, especially 400-700, such as polyethylene glycol 600 and/or polyethylene glycol 400, or propylene glycol.

9. A composition according to anyone of claims 1-8 **wherein** the surfactant or surfactants are selected from non-ionic surfactants, preferably a liquid or a waxy polyoxyethylene-fatty alcohol ether, polyoxyethylene fatty acid ester, sorbitol-fatty acid ester, POE-sorbitol-fatty-acid ester, or a self-emulsifying wax.

10. A composition according to claim 8, **wherein** said surfactant or surfactants are selected from the group consisting of sorbitol-monolaurate, sorbitol-monooleate, sorbitol-monopalmitate, sorbitol-monostearate, POE-lauryl ether, POE-stearyl ether, POE-oleyl ether, POE-cetostearyl ether, POE-castor oil, POE-hydrogenated castor oil, POE-stearic acid ester POE-sorbitol-monolaurate, POE-sorbitol-monostearate,and POE-sorbitol-monooleate.

11. A composition according to anyone of claims 1-10 **wherein** the propellant has a vapour pressure at room temperature or ambient temperature within the range of 0.8-2.5 bars, preferably 1.0-2.5 bars.

12. A composition according to claim 11 **wherein** the propellant has a boiling point within the range of from -10°C to +10°C and is preferably selected from the group consisting of alkanes and chlorinated-fluorinated alkanes, e.g. CClF₂CClF₂, CH₃CCClF₂, C₄H₁₀, and C₃H₈ + C₄H₁₀.

13. A composition according to claim 12 **wherein** the propellant is n-butane or a propane/iso-butane mixture.

14. A composition according to anyone of claims 1-13 **wherein** the pharmaceutically active ingredient is selected from the group consisting of steroids having anti-inflammatory actions, 4-aminosalicylic acid and 5-aminosalicylic acid as well as pharmaceutically acceptable salts thereof, such as their sodium salts.

15. A composition according to anyone of claims 1-14 **wherein** the ingredients are mixed in such proportions that the expanded foam will have a volume that is approximately 15-20 times the volume of the non-expanded foamable composition.

## Patentansprüche

1. Eine im wesentlichen oder vollständig nichtwässrige, schäumbare Zusammensetzung für die rektale Verabreichung und enthaltend ein flüssiges, polares Polyol oder ein Polyolgemisch, einen pharmazeutisch aktiven Bestandteil, der im Rektum oder im distalen Teil des Kolons eine lokale Wirkung ausübt, mindestens ein Schaum-stabilisierendes und emulgierendes grenzflächenaktives Mittel, wobei die Zusammensetzung innerhalb eines unter Druck stehenden Behälters zusammen mit einem Treibmittel vorliegt, dadurch **gekennzeichnet**, daß die Anteile der Komponenten in der Zusammensetzung in solchen Verhältnissen und mit solcher Menge an Treibmitteln vermischt werden, daß ein Dampfdruck von weniger als 50 Vol.-% der gesamten erhaltbaren, schaumbaren Wirkung während der ersten Sekunde, nachdem die Verabreichung rektal aus dem unter Druck stehenden Behälter verabreicht wurde, erhalten wird.

2. Zusammensetzung nach Anspruch 1, dadurch **gekennzeichnet**, daß die Zusammensetzung Additive für pharmazeutische Zusammensetzungen, beispielsweise Antioxidantien, enthält.

3. Zusammensetzung nach einem der Ansprüche 1 bis 2, dadurch **gekennzeichnet**, daß der Wassergehalt der Zusammensetzung 0 bis 30, bevorzugt 0 bis 20 und am meisten bevorzugt 0 bis 3 Gew.-%, bezogen auf das Gesamtgewicht aus Polyol und Wasser, beträgt.

4. Zusammensetzung nach einem der Ansprüche 1 bis 3, dadurch **gekennzeichnet**, daß die Zusammensetzung 60 bis 95, bevorzugt 70 bis 93 Gew.-% Polyol plus Wasser; bis zu 30, bevorzugt 0,001 bis 25 Gew.-% an pharmazeutisch aktivem Bestandteil; 0,2 bis 10, bevorzugt 0,5 bis 8 Gew.% an grenzflächenaktivem Mittel; 0 bis 5, bevorzugt 0 bis 2 Gew.-% an Additiven; und 1 bis 15, bevorzugt 2 bis 10 Gew.-% an Treibmittel enthält, wobei alle Prozentgehalte auf dem Gesamtgewicht der Zusammensetzung einschließlich des Treibmittels basieren.

5. Zusammensetzung nach Anspruch 4, dadurch **gekennzeichnet**, daß die Zusammensetzung 70 bis 89 Gew.-% Polyol plus Wasser; 5 bis 25 Gew.-% an pharmazeutisch aktivem Bestandteil; 0,8 bis 6 Gew.-% an grenzflächenaktivem Mittel; 0 bis 2 Gew.-% an Additiven; und 2 bis 10 Gew.-% an Treibmittel enthält.

6. Zusammensetzung nach Anspruch 4, dadurch **gekennzeichnet**, daß die Zusammensetzung 89 bis 93 Gew.-% Polyol plus Wasser; 0,001 bis 5 Gew.-% an pharmazeutisch aktivem Bestandteil; 0,8 bis 6 Gew.-% an grenzflächenaktivem Mittel; 0 bis 2 Gew.-% an Additiven; und 2 bis 8 Gew.-% an Treibmittel enthält.

7. Zusammensetzung nach einem der Ansprüche 1 bis 6, dadurch **gekennzeichnet**, daß die Verhältnisse der Bestandteile einschließlich des Treibmittels und der Dampfdruck davon so gewählt werden, daß weniger als 50 Vol.-% der gesamten erhaltenen Schaumwirkung während der ersten 5 Sekunden des Schäumens erhalten werden.

8. Zusammensetzung nach einem der Ansprüche 1 bis 7, dadurch **gekennzeichnet**, daß das Polyol oder die Polyole ausgewählt werden aus der Gruppe, bestehend aus flüssigen Polyalkylenglykolen, Alkylenglykolen und Glyzerin, bevorzugt Polyalkylenglykolen, insbesondere Polyethylenglykol mit einem durchschnittlichen Molekulargewicht von 300 bis 800, insbesondere 400 bis 700 wie Polyethylenglykol 600 und/oder Polyethylenglykol 400 oder Propylenglykol.

9. Zusammensetzung nach irgendeinem der Ansprüche 1 bis 8, dadurch **gekennzeichnet**, daß das grenzflächenaktive Mittel oder die grenzflächenaktiven Mittel ausgewählt werden aus nichtionischen grenzflächenaktiven Mitteln, bevorzugt einem flüssigen oder einem wachsartigen Polyoxyethylen-Fettalkohol-Ether, einem Polyoxyethylen-Fettsäure-Ester, Sorbit-Fettsäure-Ester, POE-Sorbit-Fettsäure-Ester oder einem selbstemulgierenden Wachs.

10. Zusammensetzung nach Anspruch 8, dadurch **gekennzeichnet**, daß das grenzflächenaktive Mittel oder die grenzflächenaktiven Mittel ausgewählt werden aus der Gruppe, bestehend aus Sorbit-Monolaurat, Sorbit-Monooleat, Sorbit-Monopalmitat, Sorbit-Monostearat, POE-Lauryl-Ether, POE-Stearyl-Ether, POE-Oleyl-Ether, POE-Zetostearyl-Ether, POE-Castoröl, POE-hydrogeniertes Castoröl, POE-stearines Säureester, POE-Sorbit-Monolaurat, POE-Sorbit-Monostearat, und POE-Sorbit-Monooleat.

11. Zusammensetzung nach einem der Ansprüche 1 bis 10, dadurch **gekennzeichnet**, daß das Treibmittel einen Dampfdruck bei Raumtemperatur oder Umgebungstemperatur innerhalb des Bereiches von 0,8 bis 2,5 bar, bevorzugt 1,0 bis 2,5 bar besitzt.

12. Zusammensetzung nach Anspruch 11, dadurch **gekennzeichnet**, daß das Treibmittel einen Siedepunkt innerhalb des Bereiches von -10 °C bis + 10 °C besitzt und bevorzugt ausgewählt wird aus der Gruppe, bestehend aus Alkanen und chlorierten, fluorinierten Alkanen, beispielsweise CClF₂CClF₂, CH₃CCClF₂, C₄H₁₀, und C₃H₈ + C₄H₁₀.

13. Zusammensetzung nach Anspruch 12, dadurch **gekennzeichnet**, daß das Treibmittel n-Butan oder ein Propan/ Isobutangemisch ist.

14. Zusammensetzung nach einem der Ansprüche 1 bis 13, dadurch **gekennzeichnet**, daß der pharmazeutisch aktive Bestandteil ausgewählt wird aus der Gruppe, bestehend aus Steroiden mit anti-inflammatorischen Wirkungen, 4-Aminosalicylsäure und 5-Aminosalicylsäure wie auch den pharmazeutisch annehmbaren Salzen davon, wie ihren Natriumsalzen.

15. Zusammensetzung nach einem der Ansprüche 1 bis 14, dadurch **gekennzeichnet**, daß die Bestandteile in solchen Verhältnissen gut vermischt werden, daß der expandierte Schaum ein Volumen besitzt, das das ungefähr 15 bis 20fache des Volumens der nicht-expandierbaren, schäumbaren Zusammensetzung beträgt.

## Revendications

1. Composition essentiellement ou totalement non aqueuse, apte au moussage, pour l'administration rectale, qui comprend un polyol ou mélange de polyols polaires liquides, un ingrédient pharmaceutiquement actif capable d'exercer un effet local dans le rectum ou la partie distale du colon, au moins un surfactant à effet de stabilisation de la mousse et d'émulsionnement, ladite composition étant logée à l'intérieur d'un récipient mis sous pression en association avec un agent propulseur, dans laquelle les constituants de la composition sont mélangés en des proportions telles et avec une quantité telle de l'agent propulseur ayant une pression de vapeur telle que moins de 50 % en volume de l'effet moussant pouvant être obtenu au total soient atteints au cours de la première seconde après l'administration rectale de la composition à partir du récipient mis sous pression.

2. Composition suivant la revendication 1, qui contient des additifs pour compositions pharmaceutiques, par exemple des anti-oxydants.

3. Composition suivant l'une quelconque des revendications 1 et 2, dont la teneur en eau va de 0 à 30, de préférence de 0 à 20 et d'au plus 0 à 3 % en poids du poids total du polyol et de l'eau.

4. Composition suivant l'une quelconque des revendications 1 à 3, qui comprend 60 à 95, de préférence 70 à 93 % en poids de la somme du polyol et de l'eau ; jusqu'à 30, de préférence 0,001 à 25 % en poids de l'ingrédient pharmaceutiquement actif ; 0,2 à 10, de préférence 0,5 à 8, % en poids du surfactant ; 0 à 5, de préférence 0 à 2 % en poids des additifs ; et 1 à 15, de préférence 2 à 10 % en poids de l'agent propulseur, tous les pourcentages étant basés sur le poids total de la composition comprenant l'agent propulseur.

5. Composition suivant la revendication 4, qui comprend 70 à 89 % en poids de la somme du polyol et de l'eau ; 5 à 25 % en poids de l'ingrédient pharmaceutiquement actif ; 0,8 à 6 % en poids du surfactant ; 0 à 2 % en poids des additifs ; et 2 à 10 % en poids de l'agent propulseur.

6. Composition suivant la revendication 4, qui comprend 89 à 93 % en poids de la somme du polyol et de l'eau ; 0,001 à 5 % en poids de l'ingrédient pharmaceutiquement actif ; 0,8 à 6 % en poids du surfactant ; 0 à 2 % en poids des additifs ; et 2 à 8 % en poids de l'agent propulseur.

7. Composition suivant l'une quelconque des revendications 1 à 6, dans laquelle les proportions des ingrédients comprenant l'agent propulseur et la pression de vapeur de cet agent sont choisis de manière à obtenir moins de 50 % en volume de l'effet moussant total pouvant être obtenu au cours des 5 premières secondes du moussage.

8. Composition suivant l'une quelconque des revendications 1 à 7, dans laquelle le ou les polyols sont choisis dans le groupe consistant en polyalkylèneglycols liquides, alkylèneglycols et glycérols, de préférence polyalkylèneglycols, notamment des polyéthylèneglycols, ayant un poids moléculaire moyen de 300 à 800, notamment de 400 à 700, tels que le polyéthylèneglycol 600 et/ou le polyéthylèneglycol 400, ou bien le propylèneglycol.

9. Composition suivant l'une quelconque des revendications 1 à 8, dans laquelle le ou les surfactants sont choisis parmi les surfactants non ioniques, de préférence un éther liquide ou cireux de polyoxyéthylène et d'un alcool gras, un ester de polyoxyéthylène et d'un acide gras, un ester de sorbitol et d'un acide gras, un ester de POE-sorbitol-acide gras ou une cire auto-émulsionnable.

10. Composition suivant la revendication 8, dans laquelle le ou les surfactants sont choisis dans le groupe consistant en monolaurate de sorbitol, mono-oléate de sorbitol, monopalmitate de sorbitol, monostérate de sorbitol, éther laurylique de POE, éther stéarylique de POE, éther oléylique de POE, éther cétostéarylique de POE, POE-huile de ricin, POE-huile de ricin hydrogénée, ester de POE et d'acide stéarique, POE-monolaurate de sorbitol, POE-monostéarate de sorbitol et POE-mono-oléate de sorbitol.

11. Composition suivant l'une quelconque des revendications 1 à 10, dans laquelle l'agent propulseur a une pression de vapeur à température du local ou température ambiante comprise dans l'intervalle de 0,8 à 2,5 bars, de préférence de 1,0 à 2,5 bars.

12. Composition suivant la revendication 11, dans laquelle l'agent propulseur a un point d'ébullition compris dans l'intervalle de -10°C à +10°C et est choisi de préférence dans le groupe consistant en alcanes et alcane chloréfluoré, par exemple CClF₂CClF₂, CH₃CCClF₂, C₄H₁₀ et C₃H₈ + C₄H₁₀.

13. Composition suivant la revendication 12, dans laquelle l'agent propulseur est le n-butane ou un mélange propane/isobutane.

14. Composition suivant l'une quelconque des revendications 1 à 13, dans laquelle l'ingrédient pharmaceutiquement actif est choisi dans le groupe consistant en stéroïdes ayant des actions anti-inflammatoires, acide 4-aminosalicylique et acide 5-aminosalicylique ainsi que leurs sels pharmaceutiquement acceptables, tels que leurs sels de sodium.

15. Composition suivant l'une quelconque des revendications 1 à 14, dans laquelle les ingrédients sont mélangés en des proportions telles que la mousse expansée ait un volume qui soit égal à approximativement 15-20 fois le volume de la composition apte au moussage non expansée.
